# EUROPEAN PATENT APPLICATION

(11) **EP 4 578 394 A1**
(43) Date of publication of application: **02.07.2025**
(21) Application number: 23857006.3
(22) Date of filing: 07.07.2023
(51) Int. Cl.: A61B 6/00

(54) **INFORMATION PROCESSING DEVICE, INFORMATION PROCESSING METHOD, AND PROGRAM**

(30) Priority: 22.08.2022 JP 2022132089
(71) Applicant: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2023/025353
(87) International publication number: WO 2024/042889

(57) **Abstract**

An information processing apparatus according to the present disclosure generates a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generates a second difference image representing a difference between a second low-energy image captured by irradiating the other of the left breast or the right breast with the radiation having the first energy and a second high-energy image captured by irradiating the other of the left breast or the right breast with the radiation having the second energy; determines an enhancement level of background mammary gland parenchyma of the one of the left breast or the right breast based on the first difference image; determines an enhancement level of background mammary gland parenchyma of the other of the left breast or the right breast based on the second difference image; and determines symmetry of enhancement regions of the background mammary gland parenchyma related to the left and right breasts based on a first feature value extracted from the first difference image and a second feature value extracted from the second difference image.

## Description

### BACKGROUND OF THE INVENTION

### 1. Field of the Invention

The present disclosure relates to an information processing apparatus, an information processing method, and a program.

### 2. Description of the Related Art

Contrast-enhanced mammography, which acquires a low-energy image and a high-energy image by performing imaging by irradiating a breast in which a contrast agent is injected with radiation having different energies and obtains a difference between the low-energy image and the high-energy image to generate an image in which a lesion or the like is contrast-enhanced, is known. In recent years, since the contrast-enhanced mammography has been included in a comprehensive guideline for breast cancer image diagnosis called a breast imaging reporting and data system (BI-RADS), there is a high possibility that the contrast-enhanced mammography will be widely used as a standard diagnosis method.

However, it is difficult to perform the interpretation of the image obtained by the contrast-enhanced mammography. One of the reasons for the difficulty is an effect of background mammary gland parenchymal enhancement (BPE) due to the contrast agent. The BPE represents a level of enhancement of a normal structure of a mammary gland via the contrast agent, and the visibility of the enhanced lesion greatly varies depending on the level of the BPE. Therefore, in BI-RADS, it is recommended to evaluate and describe an enhancement level of the background mammary gland parenchyma and the symmetry of enhancement regions of the background mammary gland parenchyma related to the left and right breasts in a case of the interpretation. Hereinafter, the enhancement level of the background mammary gland parenchyma will be referred to as a "BPE level", the enhancement region of the background mammary gland parenchyma will be referred to as a "BPE region", and the symmetry of the BPE regions related to the left and right breasts will be referred to as "BPE symmetry". The BPE level represents a ratio of the enhanced mammary gland parenchyma to the background mammary gland.

As described above, since the difficulty of the interpretation is high in the contrast-enhanced mammography, it is desired to support even a doctor who is not accustomed to the interpretation so that standard interpretation can be performed. For example, WO2019/104252A discloses a technology of receiving an image of an inside of a breast and automatically classifying tissues by using a neural network. The classification of the tissues includes the classification of the mammary gland parenchyma.

### SUMMARY OF THE INVENTION

However, WO2019/104252A describes that the classification of the mammary gland parenchyma is performed, but does not disclose the determination of the BPE level and the BPE symmetry. Therefore, in the technology described in WO2019/104252A, it is not possible to sufficiently support the interpretation in the contrast-enhanced mammography.

An object of the present disclosed technology is to provide an information processing apparatus, an information processing method, and a program capable of improving support for interpretation in contrast-enhanced mammography.

In order to achieve the above-described object, the present disclosure provides an information processing apparatus comprising: at least one processor, in which the processor is configured to: generate a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generate a second difference image representing a difference between a second low-energy image captured by irradiating the other of the left breast or the right breast with the radiation having the first energy and a second high-energy image captured by irradiating the other of the left breast or the right breast with the radiation having the second energy; determine an enhancement level of background mammary gland parenchyma of the one of the left breast or the right breast based on the first difference image; determine an enhancement level of background mammary gland parenchyma of the other of the left breast or the right breast based on the second difference image; and determine symmetry of enhancement regions of the background mammary gland parenchyma related to the left and right breasts based on a first feature value extracted from the first difference image and a second feature value extracted from the second difference image.

It is preferable that the processor is configured to: determine the enhancement level by inputting the first difference image to a first machine learned model; determine the enhancement level by inputting the second difference image to a second machine learned model; extract a feature value generated by the first machine learned model as the first feature value; and extract a feature value generated by the second machine learned model as the second feature value.

It is preferable that the processor is configured to: determine the symmetry by combining the first feature value and the second feature value and inputting the combined feature value to a third machine learned model.

It is preferable that the first machine learned model and the second machine learned model are the same model.

It is preferable that the processor is configured to: combine the first feature value and the second feature value in a channel direction and input the combined feature value to the third machine learned model.

It is preferable that each of the first machine learned model and the second machine learned model classifies the enhancement level into a plurality of classes.

It is preferable that the third machine learned model performs classification into two classes of symmetric and asymmetric.

It is preferable that the first machine learned model specifies the enhancement region via segmentation from the first difference image and displays the specified enhancement region on the first difference image, and the second machine learned model specifies the enhancement region via segmentation from the second difference image and displays the specified enhancement region on the second difference image.

It is preferable that the first machine learned model detects the enhancement region from the first difference image and displays a bounding box including the enhancement region on the first difference image, and the second machine learned model detects the enhancement region from the second difference image and displays a bounding box including the enhancement region on the second difference image.

It is preferable that the third machine learned model specifies an asymmetric region in which the enhancement regions are not symmetric between the first difference image and the second difference image based on the first feature value and the second feature value and displays the specified asymmetric region.

It is preferable that the processor is configured to: detect a nipple from the first difference image and the second difference image, the first low-energy image and the second low-energy image, or the first high-energy image and the second high-energy image, perform resizing of sizes of the left and right breasts shown in the first difference image and the second difference image based on coordinates of the detected nipple, and then determine the enhancement level and the enhancement region.

It is preferable that the processor is configured to: detect a length of a breast end from the first difference image and the second difference image, the first low-energy image and the second low-energy image, or the first high-energy image and the second high-energy image, perform resizing of sizes of the left and right breasts shown in the first difference image and the second difference image based on the detected length of the breast end, and then determine the enhancement level and the enhancement region.

It is preferable that the processor is configured to: determine the enhancement level by inputting the first difference image and the first low-energy image to a first machine learned model; determine the enhancement level by inputting the second difference image and the second low-energy image to a second machine learned model; extract a feature value generated by the first machine learned model as the first feature value; and extract a feature value generated by the second machine learned model as the second feature value.

It is preferable that the processor is configured to: detect first mammary gland information from the first low-energy image and/or the first high-energy image; detect second mammary gland information from the second low-energy image and/or the second high-energy image; determine the enhancement level by inputting the first difference image and the first mammary gland information to a first machine learned model; determine the enhancement level by inputting the second difference image and the second mammary gland information to a second machine learned model; extract a feature value generated by the first machine learned model as the first feature value; and extract a feature value generated by the second machine learned model as the second feature value.

It is preferable that each of the first mammary gland information and the second mammary gland information is a mammary gland region image representing a mammary gland region.

It is preferable that each of the first mammary gland information and the second mammary gland information is a mammary gland volume image representing a mammary gland volume for each pixel.

It is preferable that the processor is configured to: perform inversion processing of inverting one of the first difference image or the second difference image and then determine the enhancement level and the enhancement region.

The present disclosure provides an information processing method comprising: generating a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generating a second difference image representing a difference between a second low-energy image captured by irradiating the other of the left breast or the right breast with the radiation having the first energy and a second high-energy image captured by irradiating the other of the left breast or the right breast with the radiation having the second energy; determining an enhancement level of background mammary gland parenchyma of the one of the left breast or the right breast based on the first difference image; determining an enhancement level of background mammary gland parenchyma of the other of the left breast or the right breast based on the second difference image; and determining symmetry of enhancement regions of the background mammary gland parenchyma related to the left and right breasts based on a first feature value extracted from the first difference image and a second feature value extracted from the second difference image.

The present disclosure provides a program causing a computer to execute a process comprising: generating a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generating a second difference image representing a difference between a second low-energy image captured by irradiating the other of the left breast or the right breast with the radiation having the first energy and a second high-energy image captured by irradiating the other of the left breast or the right breast with the radiation having the second energy; determining an enhancement level of background mammary gland parenchyma of the one of the left breast or the right breast based on the first difference image; determining an enhancement level of background mammary gland parenchyma of the other of the left breast or the right breast based on the second difference image; and determining symmetry of enhancement regions of the background mammary gland parenchyma related to the left and right breasts based on a first feature value extracted from the first difference image and a second feature value extracted from the second difference image.

According to the present disclosed technology, it is possible to provide the information processing apparatus, the information processing method, and the program capable of improving the support for the interpretation in the contrast-enhanced mammography.

### BRIEF DESCRIPTION OF THE DRAWINGS

Fig. 1 is a diagram showing an example of an overall configuration of a radiation image capturing system.
Fig. 2 is a block diagram showing an example of a configuration of an information processing apparatus.
Fig. 3 is a block diagram showing an example of functions implemented by a control unit of the information processing apparatus.
Fig. 4 is a flowchart schematically showing a flow of contrast-enhanced imaging processing.
Fig. 5 is a flowchart schematically showing a flow of determination processing.
Fig. 6 is a diagram schematically showing an inversion processing unit.
Fig. 7 is a diagram schematically showing BPE determination processing via a BPE determination processing unit.
Fig. 8 is a diagram showing an example of determination results of a BPE level and BPE symmetry.
Fig. 9 is a diagram conceptually showing an example of a configuration of a first machine learned model.
Fig. 10 is a diagram conceptually showing combination processing of a first feature map and a second feature map.
Fig. 11 is a diagram schematically showing a flow of BPE determination processing according to a first modification example.
Fig. 12 is a diagram conceptually showing combination processing according to the first modification example.
Fig. 13 is a diagram schematically showing BPE determination processing according to a second modification example.
Fig. 14 is a diagram schematically showing BPE determination processing according to a third modification example.
Fig. 15 is a diagram schematically showing BPE determination processing according to a fourth modification example.
Fig. 16 is a block diagram showing functions implemented by a control unit according to a fifth modification example.
Fig. 17 is a diagram conceptually showing resize processing via a resize processing unit.
Fig. 18 is a diagram conceptually showing resize processing in a case in which an X coordinate and a Y coordinate are different between a first difference image and a second difference image.
Fig. 19 is a diagram conceptually showing resize processing according to a sixth modification example.
Fig. 20 is a diagram schematically showing BPE determination processing according to a seventh modification example.
Fig. 21 is a block diagram showing functions implemented by a control unit according to an eighth modification example.
Fig. 22 is a diagram conceptually showing mammary gland information detection processing via a mammary gland information detection processing unit.
Fig. 23 is a diagram conceptually showing mammary gland information detection processing according to a ninth modification example.
Fig. 24 is a diagram showing an example in which only the BPE level of one of a left breast or a right breast is determined.

### DESCRIPTION OF THE PREFERRED EMBODIMENTS

Hereinafter, an embodiment of the present disclosure will be described in detail with reference to the accompanying drawings.

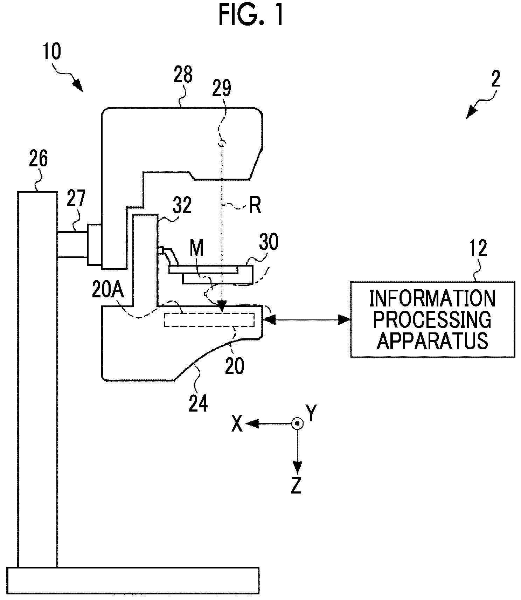

Fig. 1 shows an example of an overall configuration of a radiation image capturing system 2 according to the present embodiment. The radiation image capturing system 2 comprises a mammography apparatus 10 and an information processing apparatus 12. The information processing apparatus 12 is connected to a radiology information system (RIS), a picture archiving and communication system (PACS), and the like (none of which is shown) via a network or the like.

Fig. 1 shows an example of an appearance of the mammography apparatus 10. It should be noted that Fig. 1 shows an example of the appearance in a case in which the mammography apparatus 10 is seen from a left side of a person under an examination.

The mammography apparatus 10 is a radiography apparatus that operates under the control of the information processing apparatus 12 and that irradiates a breast M of a person under an examination, as a subject, with radiation R (for example, X-rays) from a radiation source 29 to capture a radiation image of the breast M.

As shown in Fig. 1, the mammography apparatus 10 comprises an imaging table 24, a base 26, an arm part 28, and a compression unit 32. A radiation detector 20 is disposed inside the imaging table 24. As shown in Fig. 1, in a case in which imaging is performed, in the mammography apparatus 10, the breast M of the person under an examination is positioned on the imaging table 24 by a user, such as a radiologist.

The radiation detector 20 detects the radiation R passing through the breast M as a subject. Specifically, the radiation detector 20 detects the radiation R passing through the breast M of the person under an examination, entering into the imaging table 24, and reaching a detection surface 20A of the radiation detector 20, and generates a radiation image based on the detected radiation R. The radiation detector 20 outputs image data representing the generated radiation image. Hereinafter, the series of operations of irradiating the breast with the radiation R from the radiation source 29 to generate the radiation image via the radiation detector 20 may be referred to as "imaging". The radiation detector 20 may be an indirect conversion type radiation detector that converts the radiation R into light beams and converts the converted light beams into charges, or may be a direct conversion type radiation detector that directly converts the radiation R into charges.

Hereinafter, two directions orthogonal to each other and parallel to the detection surface 20A will be referred to as an X direction and a Y direction. In addition, a direction orthogonal to the X direction and the Y direction will be referred to as a Z direction.

A compression plate 30 that is used for compressing the breast M in a case of performing the imaging is attached to the compression unit 32. The compression plate 30 is moved in a direction approaching or in a direction spaced away from the imaging table 24 by a compression plate drive unit (not shown) provided in the compression unit 32. The compression plate 30 is moved in a direction approaching the imaging table 24 to compress the breast M with the imaging table 24.

The arm part 28 can be rotated with respect to the base 26 by a shaft part 27. The shaft part 27 is fixed to the base 26, and the shaft part 27 and the arm part 28 are rotated integrally. Gears are provided in each of the shaft part 27 and the compression unit 32 of the imaging table 24, and the gears are switched between an engaged state and a non-engaged state, so that a state in which the compression unit 32 of the imaging table 24 and the shaft part 27 are connected to each other and are rotated integrally and a state in which the shaft part 27 is separated from the imaging table 24 and idles can be switched. The elements for switching between transmission and non-transmission of power of the shaft part 27 are not limited to the gears, and various mechanical elements can be used. The arm part 28 and the imaging table 24 can be separately rotated relative to the base 26 with the shaft part 27 as a rotation axis.

The mammography apparatus 10 can perform the imaging on each of the left and right breasts M from a plurality of directions by rotating the arm part 28. For example, it is possible to perform cranio-caudal (CC) imaging and medio-lateral oblique (MLO) imaging.

The radiation image capturing system 2 can perform "contrast-enhanced imaging" in which the imaging is performed in a state in which a contrast agent is injected in the breast M. Specifically, the radiation image capturing system 2 has a contrast enhanced digital mammography (CEDM) function of performing contrast enhancement via energy subtraction.

In the contrast-enhanced imaging, a low-energy image and a high-energy image are acquired by performing the imaging by irradiating the breast M, in which the contrast agent is injected, with the radiation R having different energies. In the present disclosure, a radiation image captured by the radiation R having a first energy will be referred to as a "low-energy image", and a radiation image captured by the radiation R having a second energy higher than the first energy will be referred to as a "high-energy image". Hereinafter, in a case in which the low-energy image and the high-energy image are not distinguished from each other, the low-energy image and the high-energy image will be simply referred to as a radiation image.

In the contrast-enhanced imaging, for example, an iodine contrast agent having a k absorption edge of 32 keV is used as the contrast agent. In the contrast-enhanced imaging in a case in which the iodine contrast agent is used, the first energy need only be set to be lower than the k absorption edge, and the second energy need only be set to be higher than the k absorption edge.

The contrast agent and the body tissue such as the mammary gland are different in absorption characteristics of the radiation R. Therefore, the high-energy image clearly shows the contrast agent in addition to the body tissue such as the mammary gland and the fat. On the other hand, in the low-energy image, the body tissue is clearly shown, but the contrast agent is hardly shown. Therefore, by taking a difference between the low-energy image and the high-energy image, it is possible to generate a difference image in which the mammary gland structure is erased and a lesion or the like stained with the contrast agent is enhanced. The lesion consists of, for example, new cells and is easily stained with the contrast agent.

The mammography apparatus 10 and the information processing apparatus 12 are connected by wired communication or wireless communication. The radiation image generated by the radiation detector 20 in the mammography apparatus 10 is output to the information processing apparatus 12 by wired communication or wireless communication via a communication interface (I/F) (not shown).

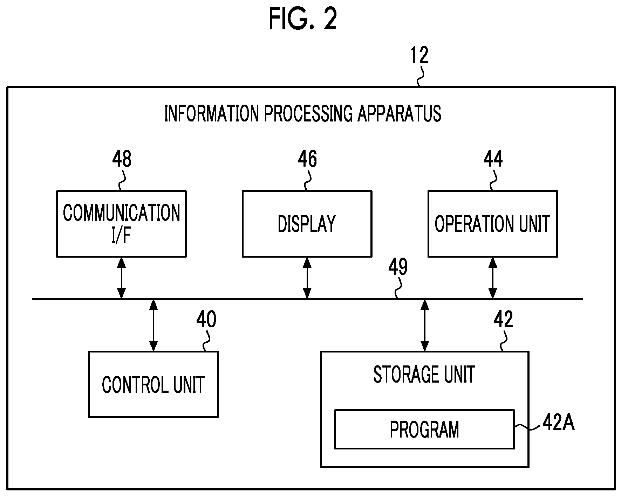

Fig. 2 shows an example of the configuration of the information processing apparatus 12. The information processing apparatus 12 comprises a control unit 40, a storage unit 42, an operation unit 44, a display 46, and a communication I/F 48. The control unit 40, the storage unit 42, the operation unit 44, the display 46, and the communication I/F 48 are connected to each other via a bus 49 such that various kinds of information can be exchanged.

The control unit 40 controls an overall operation of the radiation image capturing system 2. The control unit 40 is configured by, for example, a computer comprising a central processing unit (CPU), a read only memory (ROM), and a random access memory (RAM).

The storage unit 42 stores information related to radiography, the radiation image acquired from the mammography apparatus 10, and the like. In addition, the storage unit 42 stores a program 42A for the control unit 40 to perform various kinds of information processing described later and data for constructing various kinds of machine learned models described later. The storage unit 42 is, for example, a nonvolatile storage device such as a hard disk drive (HDD) or a solid state drive (SSD).

The operation unit 44 includes input devices such as various buttons, switches, a touch panel, a touch pen, and a mouse, which are operated by the user. The display 46 displays information related to the imaging, a radiation image obtained by the imaging, a determination result of BPE determination described later, and the like.

The communication I/F 48 performs communication of various kinds of data, such as information related to the radiography and the radiation image, with the mammography apparatus 10, the RIS, the PACS, and the like via wired communication or wireless communication.

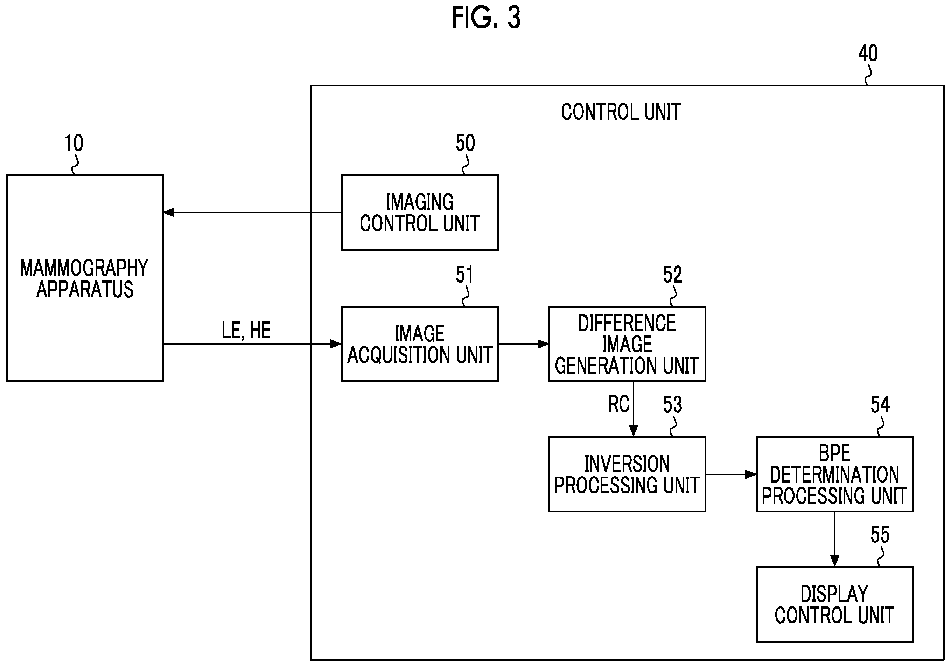

Fig. 3 shows an example of functions implemented by the control unit 40 of the information processing apparatus 12. The control unit 40 implements various functions by executing the processing based on the program 42A stored in the storage unit 42. The control unit 40 functions as an imaging control unit 50, an image acquisition unit 51, a difference image generation unit 52, an inversion processing unit 53, a BPE determination processing unit 54, and a display control unit 55.

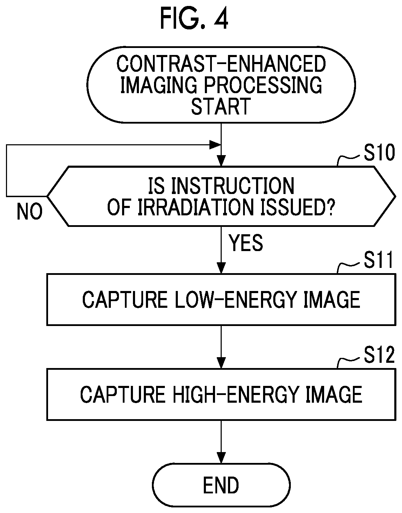

Fig. 4 schematically shows a flow of contrast-enhanced imaging processing. Processing via the imaging control unit 50 will be described with reference to Fig. 4.

First, before the imaging via the mammography apparatus 10 is started, the user, such as the radiologist, injects the contrast agent into one of the left breast M or the right breast M of the person under an examination, positions the breast M in which the contrast agent is injected on the imaging table 24, and compresses the breast M with the compression plate 30.

In step S10, the imaging control unit 50 determines whether or not an instruction of the irradiation with the radiation R is received. In a case in which the instruction of the irradiation is received, the imaging control unit 50 outputs, in step S11, an instruction of the irradiation with the radiation R having the first energy to the mammography apparatus 10. In the mammography apparatus 10, a low-energy image LE is captured by emitting the first energy radiation R toward the breast M.

In next step S12, the imaging control unit 50 outputs an instruction of the irradiation with the radiation R having the second energy to the mammography apparatus 10. In the mammography apparatus 10, a high-energy image HE is captured by emitting the radiation R having the second energy toward the breast M. It should be noted that the high-energy image HE may be captured earlier than the low-energy image LE.

In a case in which the capturing of the low-energy image LE and the high-energy image HE of the breast M ends, the user releases the compression of the breast M for which the imaging ends. Then, the user injects the contrast agent into the other breast M, positions the breast M in which the contrast agent is injected on the imaging table 24, and compresses the breast M with the compression plate 30. Thereafter, the imaging control unit 50 performs the contrast-enhanced imaging processing (steps S10 to S12) on the other breast M.

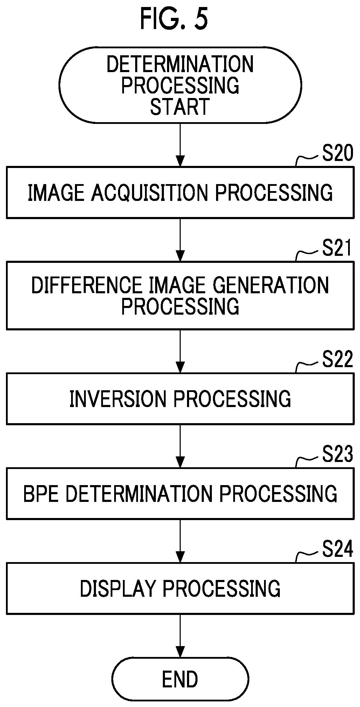

Fig. 5 schematically shows a flow of determination processing. Processing via the image acquisition unit 51, the difference image generation unit 52, the inversion processing unit 53, the BPE determination processing unit 54, and the display control unit 55 will be described with reference to Fig. 5.

In step S20, the image acquisition unit 51 acquires the low-energy image LE and the high-energy image HE captured by the above-described contrast-enhanced imaging processing for each of the left and right breasts M. Hereinafter, the low-energy image LE and the high-energy image HE for the left breast M will be referred to as a first low-energy image LE1 and a first high-energy image HE1, respectively. In addition, the low-energy image LE and the high-energy image HE for the right breast M will be referred to as a second low-energy image LE2 and a second high-energy image HE2, respectively.

In next step S21, the difference image generation unit 52 generates a difference image RC representing a difference between the low-energy image LE and the high-energy image HE for each of the left and right breasts M. For example, the difference image generation unit 52 generates the difference image RC by subtracting an image obtained by multiplying the low-energy image LE by a predetermined coefficient from an image obtained by multiplying the high-energy image HE by a predetermined coefficient for each corresponding pixel. Hereinafter, the difference image RC representing the difference between the first low-energy image LE1 and the first high-energy image HE1 will be referred to as a "first difference image RC1", and the difference image RC representing the difference between the second low-energy image LE2 and the second high-energy image HE2 will be referred to as a "second difference image RC2".

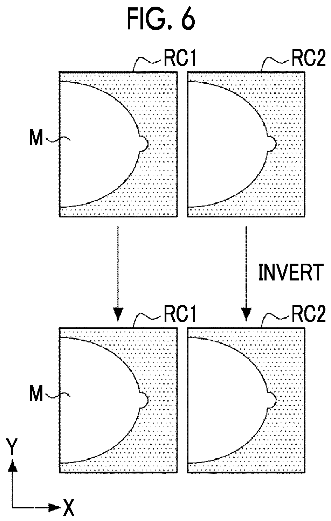

In next step S22, the inversion processing unit 53 inverts one of the first difference image RC1 and the second difference image RC2. For example, as shown in Fig. 6, the inversion processing unit 53 inverts the second difference image RC2 in the Y direction. This is because a shape of the left breast M shown in the first difference image RC1 and a shape of the right breast M shown in the second difference image RC2 have a line-symmetrical relationship with respect to an axis parallel to the X direction.

In next step S23, the BPE determination processing unit 54 performs BPE determination processing, which will be described later, by using the first difference image RC1 and the second difference image RC2 on which the inversion processing is performed. The BPE determination processing includes a determination of a BPE level and a determination of a BPE symmetry.

In next step S24, the display control unit 55 displays the determination results of the BPE level and the BPE symmetry on the display 46. The display control unit 55 may display the first difference image RC1 and the second difference image RC2 on the display 46 along with the determination results of the BPE level and the BPE symmetry. In addition, the display control unit 55 may display the first low-energy image LE1 and the second low-energy image LE2 on the display 46 along with the determination results of the BPE level and the BPE symmetry. Further, the display control unit 55 may display the first high-energy image HE1 and the second high-energy image HE2 on the display 46 along with the determination results of the BPE level and the BPE symmetry.

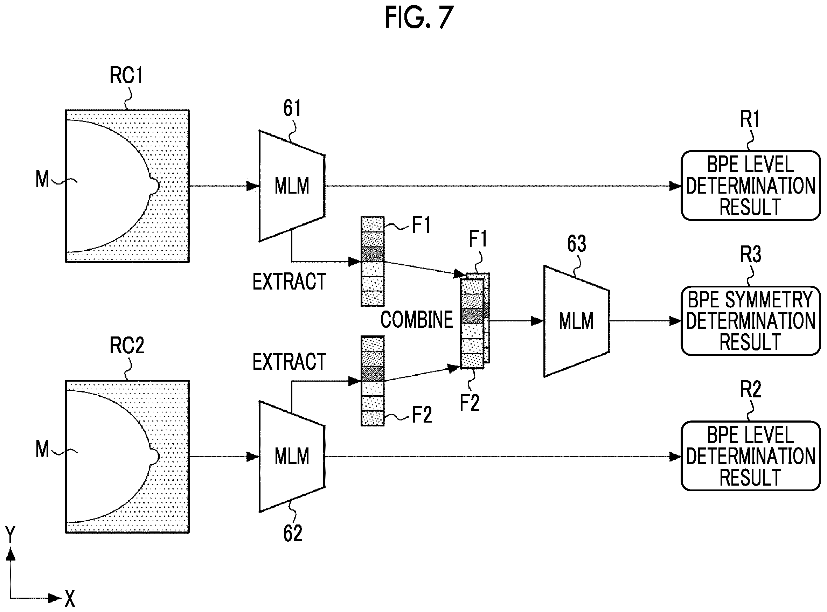

Fig. 7 schematically shows the BPE determination processing via the BPE determination processing unit 54. The BPE determination processing unit 54 determines the BPE level of the left breast M by inputting the first difference image RC1 to a first machine learned model (MLM) 61 that functions as a first BPE level determination unit. The first MLM 61 outputs a determination result R1 of the BPE level for the left breast M.

The BPE determination processing unit 54 determines the BPE level of the right breast M by inputting the second difference image RC2 to a second MLM 62 that functions as a second BPE level determination unit. The second MLM 62 outputs a determination result R2 of the BPE level for the right breast M.

In the present embodiment, each of the first MLM 61 and the second MLM 62 is configured by a convolutional neural network (CNN). The first MLM 61 and the second MLM 62 are the same model. It should be noted that the same model refers to a machine learned model having the same configuration, which is obtained by performing machine learning using the same training data.

In addition, the BPE determination processing unit 54 acquires a first feature map F1 from the first MLM 61 and acquires a second feature map F2 from the second MLM 62. The first feature map F1 is an example of a "first feature value" according to the present disclosed technology. The second feature map F2 is an example of a "second feature value" according to the present disclosed technology.

The BPE determination processing unit 54 combines the first feature map F1 and the second feature map F2 and inputs the combined first feature map F1 and second feature map F2 to a third MLM 63 that functions as a BPE symmetry determination unit, to determine the BPE symmetry of the left and right breasts M. The third MLM 63 is configured by, for example, a CNN, similarly to the first MLM 61 and the second MLM 62.

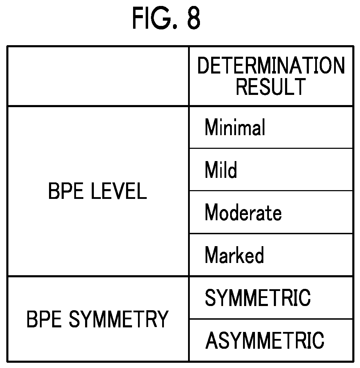

Fig. 8 shows an example of the determination results of the BPE level and the BPE symmetry. As shown in Fig. 8, the determination results of the BPE level are classified into four classes of "Minimal", "Mild", "Moderate", and "Marked". A ratio of the contrast-enhanced mammary gland parenchyma to the background mammary gland is "Minimal" in a case of being less than 25%, "Mild" in a case of being 25% or more and less than 50%, "Moderate" in a case of being 50% or more and less than 75%, and "Marked" in a case of being 75% or more. In "Marked", there is a possibility that the lesion is buried in the background mammary gland and the lesion is invisible.

The first MLM 61 outputs, as the determination result R1, a class to which the BPE level of the left breast M belongs among the four classes. The second MLM 62 outputs, as the determination result R2, a class to which the BPE level of the right breast M belongs among the four classes.

As shown in Fig. 8, the determination results of the BPE symmetry are classified into two classes of "symmetric" and "asymmetric". The third MLM 63 outputs, as the determination result R3, a class to which the BPE symmetry of the left and right breasts M belongs.

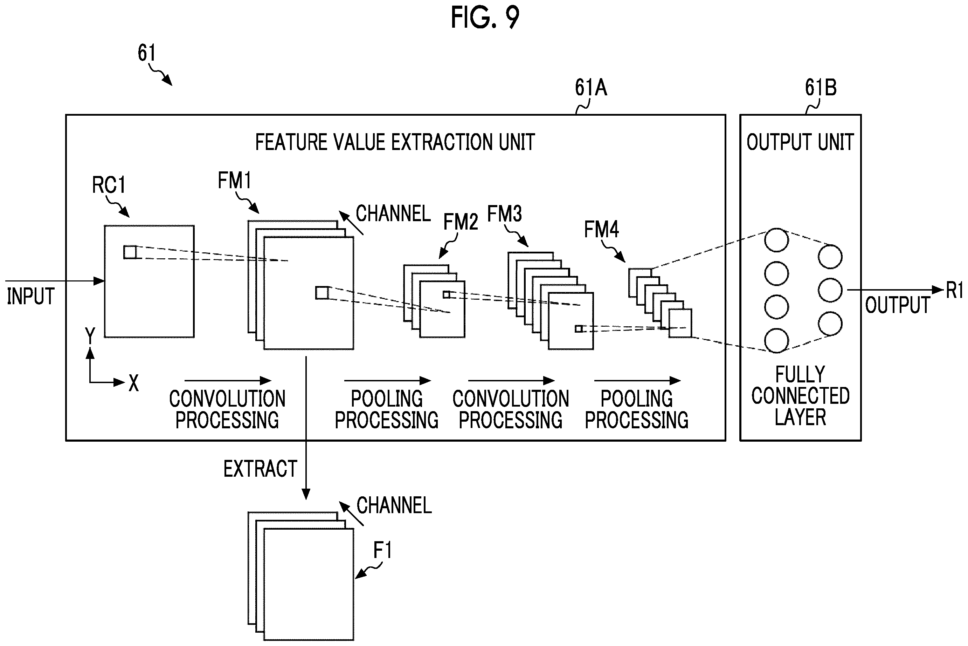

Fig. 9 conceptually shows an example of the configuration of the first MLM 61. The first MLM 61 includes a feature value extraction unit 61A and an output unit 61B. The feature value extraction unit 61A is an intermediate layer including a plurality of convolutional layers and a plurality of pooling layers. In the present embodiment, the output unit 61B is an output layer including a fully connected layer.

The first difference image RC1 is input to the feature value extraction unit 61A. The feature value extraction unit 61A extracts a feature value by executing convolution processing and pooling processing on the input first difference image RC1. In the example shown in Fig. 9, the feature value extraction unit 61 A generates a three-channel feature map FM1 by executing the convolution processing on the first difference image RC1, and generates a three-channel feature map FM2 having a reduced size by executing the pooling processing on the generated feature map FM1. Then, the feature value extraction unit 61A generates a six-channel feature map FM3 by executing the convolution processing on the feature map FM2, and generates a six-channel feature map FM4 having a reduced size by executing the pooling processing on the generated feature map FM3. The number of channels is determined by the number of filters used in a case in which the convolution processing is performed.

The output unit 61B performs the class classification based on the feature value extracted by the feature value extraction unit 61A, and outputs a result of the class classification as the determination result R1. In the example shown in Fig. 9, the feature map FM4 is input from the feature value extraction unit 61A to the output unit 61B. The output unit 61B performs the class classification based on the feature map FM4.

The BPE determination processing unit 54 extracts any one of the feature map FM1, the feature map FM2, the feature map FM3, or the feature map FM4 generated by the feature value extraction unit 61A as the first feature map F1. In the example shown in the figure, the BPE determination processing unit 54 extracts the feature map FM1 corresponding to a first convolutional layer as the first feature map F1.

The second MLM 62 has the same configuration as the first MLM 61. The BPE determination processing unit 54 extracts the feature maps corresponding to the same convolutional layer or pooling layer from the first MLM 61 and the second MLM 62, as the first feature map F1 and the second feature map F2. That is, the BPE determination processing unit 54 extracts the feature maps having the same size and the same number of channels from the first MLM 61 and the second MLM 62, as the first feature map F1 and the second feature map F2.

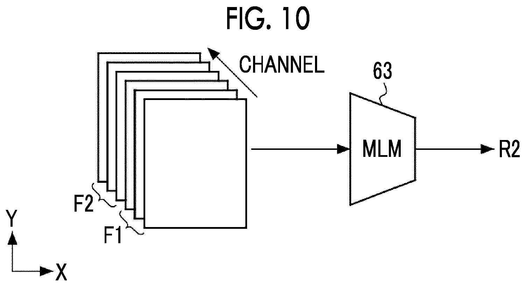

Fig. 10 conceptually shows combination processing of the first feature map F1 and the second feature map F2. In the present embodiment, the BPE determination processing unit 54 combines the first feature map F1 and the second feature map F2 in a channel direction. Therefore, in the present embodiment, the BPE determination processing unit 54 combines the first feature map F1 and the second feature map F2 in a superimposed manner in the channel direction. That is, in the present embodiment, an overall size of the combined first feature map F1 and second feature map F2 is the same as each size of the first feature map F1 and the second feature map F2, but the number of channels is doubled.

The third MLM 63 has the same configuration as the first MLM 61. The third MLM 63 performs the extraction of the feature value and the class classification by using the combined first feature map F1 and second feature map F2 as input, and outputs the result of the class classification as the determination result R2.

In a training phase, the third MLM 63 is generated by training the learning model through machine learning using a feature value obtained by combining the two feature values in the channel direction and ground-truth data of the BPE symmetry.

As described above, in the present embodiment, the BPE level of the left and right breasts M is determined by the first MLM 61 and the second MLM 62, and the BPE symmetry is determined based on the feature values extracted from the first MLM 61 and the second MLM 62. Therefore, a doctor can easily and accurately determine the BPE level and the BPE symmetry, and the support for the interpretation in the contrast-enhanced mammography is improved.

In addition, in the present embodiment, the BPE symmetry is determined based on the feature values (the first feature map F1 and the second feature map F2) extracted from the first MLM 61 and the second MLM 62, instead of the first difference image RC1 and the second difference image RC2. Since this feature value has low position sensitivity and high robustness against misregistration, the BPE symmetry can be accurately determined.

In addition, as in the present embodiment, the features of the left and right breasts M are associated by combining the feature value extracted from the first MLM 61 and the feature value extracted from the second MLM 62 in the channel direction, so that the BPE symmetry can be determined with higher accuracy.

Hereinafter, various modification examples of the above-described embodiment will be described.

### [First Modification Example]

The first modification example is different from the above-described embodiment only in the BPE determination processing via the BPE determination processing unit 54. In the present modification example, the BPE determination processing unit 54 combines the feature value extracted from the first MLM 61 and the feature value extracted from the second MLM 62 for each channel.

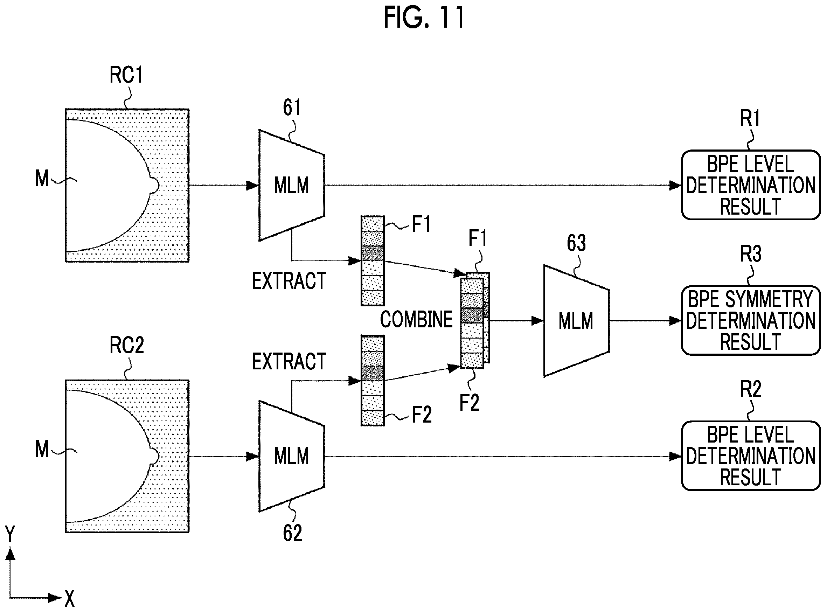

Fig. 11 schematically shows a flow of the BPE determination processing according to the first modification example. In the present modification example, the BPE determination processing unit 54 combines the first feature map F1 and the second feature map F2 for each channel, and inputs the combined feature map to the third MLM 63. In the BPE determination processing according to the first modification example, the processing other than the combination processing is the same as in the BPE determination processing according to the above-described embodiment.

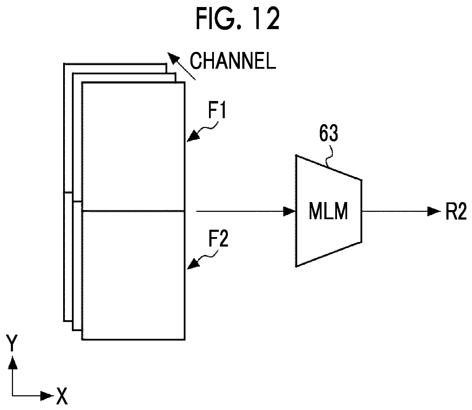

Fig. 12 conceptually shows the combination processing according to the first modification example. In the present modification example, the BPE determination processing unit 54 combines the first feature map F1 with the second feature map F2 for each channel. Therefore, in the present embodiment, the overall size of the combined first feature map F1 and second feature map F2 is twice each size of the first feature map F1 and the second feature map F2. In the example shown in Fig. 12, the first feature map F1 and the second feature map F2 are combined in a column direction (Y direction). It should be noted that the first feature map F1 and the second feature map F2 may be combined in a row direction (X direction).

In the training phase, the third MLM 63 is generated by training the learning model through machine learning using the feature value obtained by combining the two feature values for each channel and the ground-truth data of the BPE symmetry.

### [Second Modification Example]

The second modification example is different from the above-described embodiment only in the BPE determination processing via the BPE determination processing unit 54. In the present modification example, the first MLM 61 and the second MLM 62 specify a BPE region by performing segmentation based on the first difference image RC1 and the second difference image RC2.

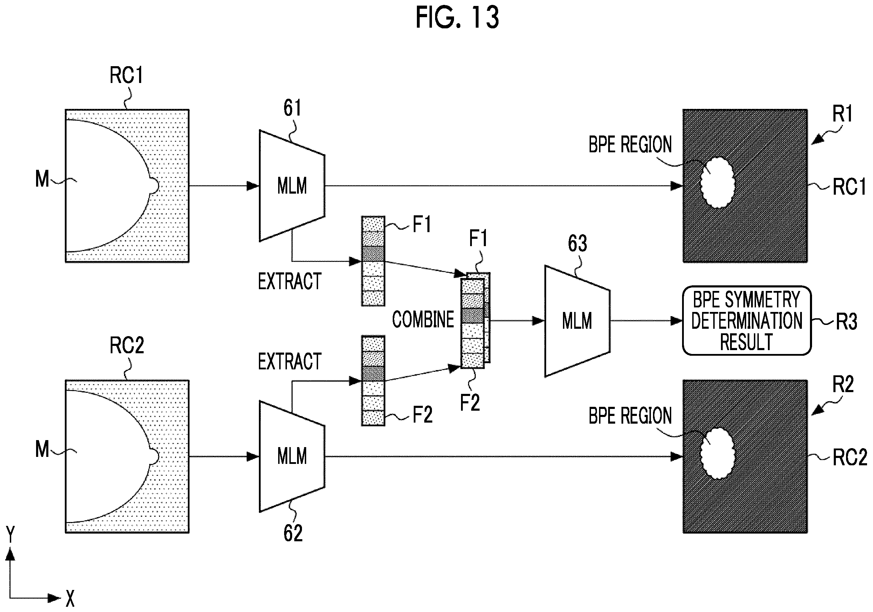

Fig. 13 schematically shows the BPE determination processing according to the second modification example. In the present modification example, for example, each of the first MLM 61 and the second MLM 62 is configured by a U-net, which is one kind of a CNN, and each pixel of the image is classified into the classes for the BPE level.

The first MLM 61 specifies the BPE region corresponding to a predetermined BPE level, and displays the BPE region on the first difference image RC1. Similarly, the second MLM 62 specifies the BPE region corresponding to the predetermined BPE level, and displays the BPE region on the second difference image RC2. In the present modification example, the first MLM 61 outputs the first difference image RC1 in which the BPE region is specified, as the determination result R1 of the BPE level. Similarly, the second MLM 62 outputs the second difference image RC2 in which the BPE region is specified, as the determination result R2 of the BPE level.

The BPE region shown in Fig. 13 is, for example, the region classified as "Marked". In the BPE determination processing according to the second modification example, processing other than the BPE level determination processing is the same as in the BPE determination processing according to the above-described embodiment.

In the example shown in Fig. 13, two regions of the BPE region and the other region are displayed in a distinguished manner, but a plurality of BPE regions may be displayed in accordance with the BPE level. For example, regions corresponding to the BPE levels of "Minimal", "Mild", "Moderate", and "Marked" may be displayed in a distinguished manner.

### [Third Modification Example]

The third modification example is different from the above-described embodiment only in the BPE determination processing via the BPE determination processing unit 54. In the present modification example, the first MLM 61 and the second MLM 62 detect the BPE region from the first difference image RC1 and the second difference image RC2.

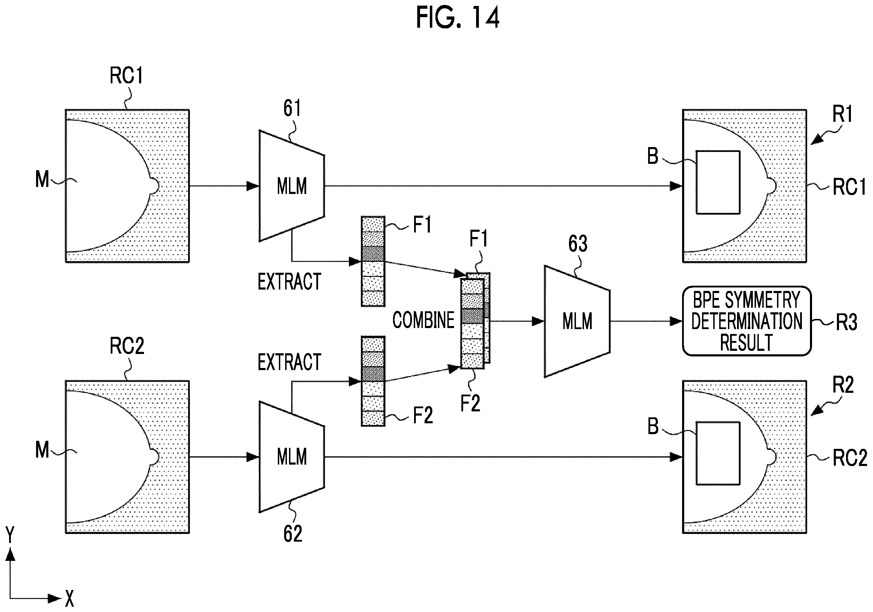

Fig. 14 schematically shows the BPE determination processing according to the third modification example. In the present modification example, for example, each of the first MLM 61 and the second MLM 62 is configured by an R-CNN (regions with CNN features), which is one kind of a CNN, and performs the detection of the object.

The first MLM 61 detects the BPE region corresponding to the predetermined BPE level, and displays a rectangular bounding box B including the detected BPE region on the first difference image RC1. Similarly, the second MLM 62 detects the BPE region corresponding to the predetermined BPE level, and displays the rectangular bounding box B including the detected BPE region on the second difference image RC2. In the present modification example, the first MLM 61 outputs the first difference image RC1 on which the bounding box B is displayed, as the determination result R1 of the BPE level. Similarly, the second MLM 62 outputs the second difference image RC2 on which the bounding box B is displayed, as the determination result R2 of the BPE level.

The bounding box B shown in Fig. 14 is, for example, the frame including a region classified as "Marked". In the BPE determination processing according to the third modification example, processing other than the BPE level determination processing is the same as in the BPE determination processing according to the above-described embodiment.

In the example shown in Fig. 14, one bounding box B is displayed, but a plurality of bounding boxes may be displayed in accordance with the BPE level. For example, the plurality of bounding boxes may be displayed to distinguish the regions corresponding to the respective BPE levels of "Minimal", "Mild", "Moderate", and "Marked".

### [Fourth Modification Example]

The fourth modification example is different from the above-described embodiment only in the BPE determination processing via the BPE determination processing unit 54. In the present modification example, the third MLM 63 specifies a BPE asymmetric region by performing segmentation based on the combined first feature map F1 and second feature map F2. The BPE asymmetric region is a region in which the BPE regions are not symmetric between the first difference image RC1 and the second difference image RC2.

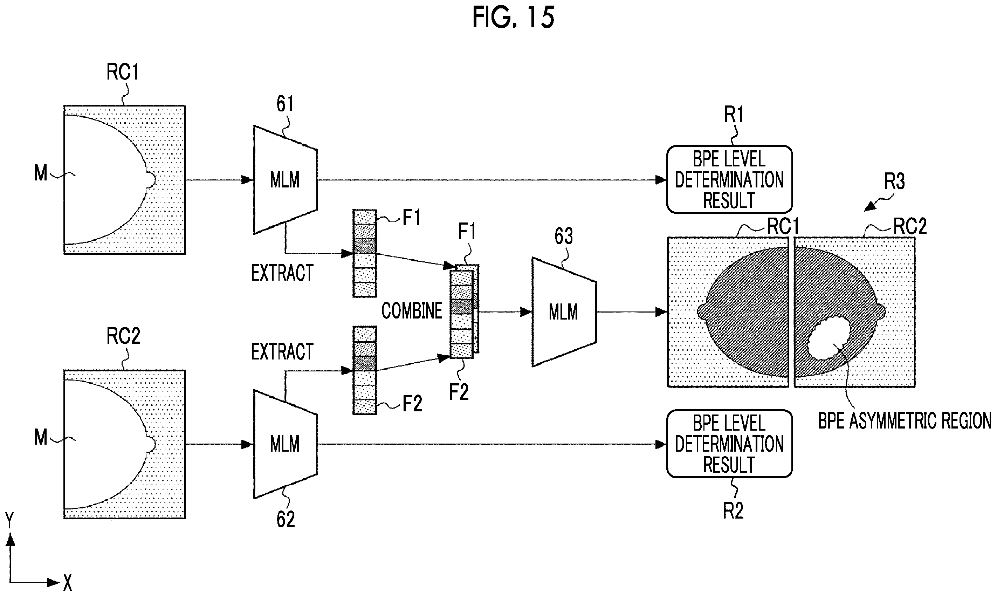

Fig. 15 schematically shows the BPE determination processing according to the fourth modification example. In the present modification example, for example, the third MLM 63 is configured by a U-net, and performs the class classification of each pixel of the image for the BPE level. The third MLM 63 specifies a region represented by pixels having different classes between the first difference image RC1 and the second difference image RC2, as the BPE asymmetric region.

In the present modification example, the third MLM 63 outputs the first difference image RC1 and the second difference image RC2 on which the BPE asymmetric region is displayed, as the determination result R3 of the BPE symmetry.

The BPE asymmetric region shown in Fig. 15 is, for example, a region that is left-right asymmetric with respect to the BPE region classified as "Marked". In the BPE determination processing according to the fourth modification example, processing other than the BPE symmetry determination processing is the same as in the BPE determination processing according to the above-described embodiment.

In the example shown in Fig. 15, one BPE asymmetric region is displayed, but a plurality of BPE asymmetric regions may be displayed in accordance with the BPE level. For example, the plurality of BPE asymmetric regions may be displayed to distinguish the symmetry of the regions corresponding to "Minimal", "Mild", "Moderate", and "Marked".

Further, as in the third modification example, the third MLM 63 may be configured to detect the BPE asymmetric region from the combined first feature map F1 and second feature map F2, and display the bounding box including the BPE asymmetric region.

### [Fifth Modification Example]

The fifth modification example is different from the above-described embodiment in that resize processing is performed on at least one of the first difference image RC1 or the second difference image RC2 before the BPE determination processing.

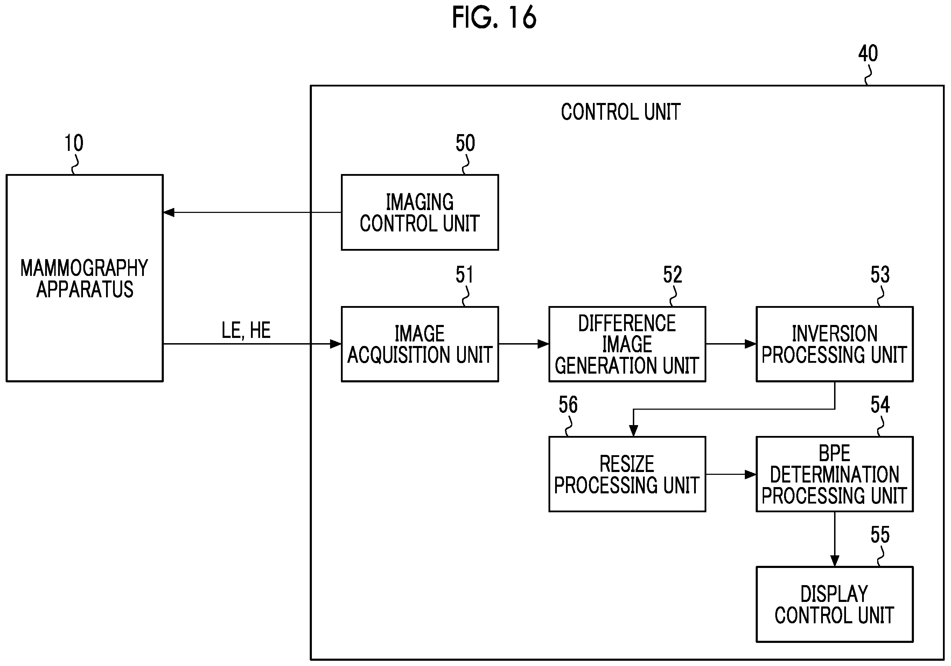

Fig. 16 shows functions implemented by the control unit 40 according to the fifth modification example. The control unit 40 according to the fifth modification example functions as a resize processing unit 56 in addition to the imaging control unit 50, the image acquisition unit 51, the difference image generation unit 52, the inversion processing unit 53, the BPE determination processing unit 54, and the display control unit 55. The control unit 40 performs the resize processing before performing the BPE determination processing after performing the inversion processing.

The resize processing unit 56 detects a nipple and performs the resize processing of resizing the size of the breast M shown in the first difference image RC1 or the second difference image RC2 based on coordinates of the detected nipple. In the present modification example, the BPE determination processing unit 54 performs the BPE determination processing by using the first difference image RC1 and the second difference image RC2 in which the size of the breast M is resized.

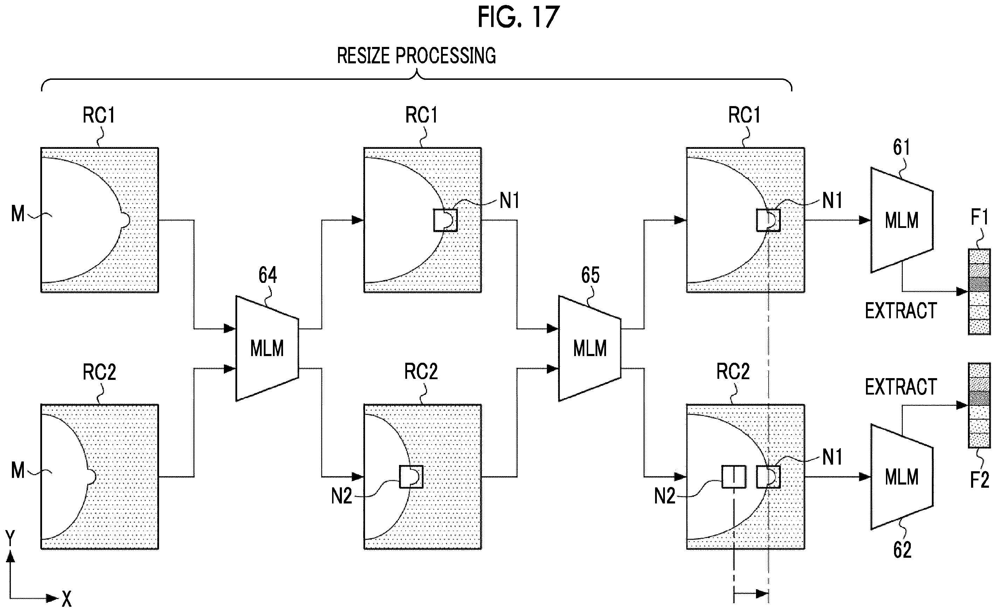

Fig. 17 conceptually shows the resize processing via the resize processing unit 56. The resize processing unit 56 detects a region including the nipple (hereinafter, referred to as a nipple region) from each of the first difference image RC1 and the second difference image RC2 by inputting the first difference image RC1 and the second difference image RC2 to a fourth MLM 64 that functions as a nipple detection unit. The fourth MLM 64 is configured by, for example, an R-CNN and detects the object. The fourth MLM 64 has been trained through machine learning using training data to detect the region including the nipple. N1 shown in Fig. 17 indicates the nipple region detected from the first difference image RC1. N2 indicates the nipple region detected from the second difference image RC2.

Next, the resize processing unit 56 deforms the first difference image RC1 or the second difference image RC2 such that the X coordinates of the nipple region N1 and the nipple region N2 match each other by inputting the first difference image RC1 including the information on the nipple region N1 and the second difference image RC2 including the information on the nipple region N2 to a fifth MLM 65 that functions as an image deformation unit. In the example shown in Fig. 17, the fifth MLM 65 deforms the second difference image RC2 such that the X coordinates of the nipple region N1 and the nipple region N2 match each other, and outputs the second difference image RC2 after the deformation and the first difference image RC1. For example, the fifth MLM 65 is configured by a CNN.

The first MLM 61 and the second MLM 62 of the BPE determination processing unit 54 are respectively input with the first difference image RC1 and the second difference image RC2 on which the resize processing is performed.

In a case in which there is a difference in the size of the left and right breasts M or a difference in the positioning of the left and right breasts M with respect to the imaging table 24, the sizes of the breasts M shown in the first difference image RC1 and the second difference image RC2 may be significantly different. In such a case, it is assumed that the determination accuracy of the BPE symmetry is decreased in a case in which the BPE determination processing is performed without performing the resize processing. In the present modification example, since the BPE determination processing is performed based on the first difference image RC1 and the second difference image RC2 on which the resize processing is performed, the determination accuracy of the BPE symmetry is improved.

Fig. 17 shows the resize processing in a case in which the X coordinate of the nipple is different between the first difference image RC1 and the second difference image RC2. The resize processing can be performed in the same manner even in a case in which the X coordinate and the Y coordinate are different between the first difference image RC1 and the second difference image RC2.

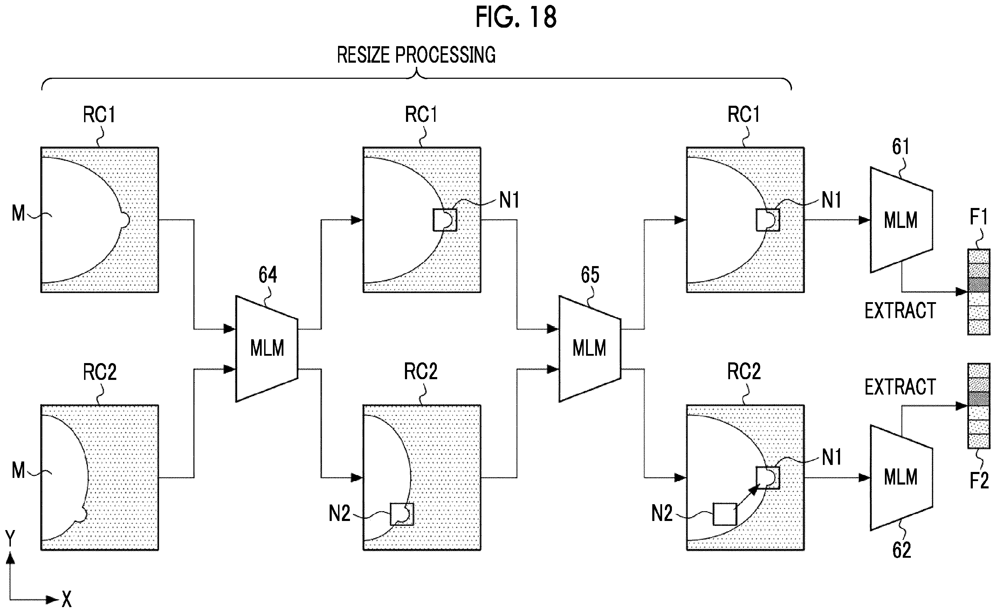

Fig. 18 conceptually shows the resize processing in a case in which the X coordinate and the Y coordinate are different between the first difference image RC1 and the second difference image RC2. In this case, the fifth MLM 65 elastically deforms the first difference image RC1 or the second difference image RC2 such that the X coordinates and the Y coordinates of the nipple region N1 and the nipple region N2 match each other. In the example shown in Fig. 18, the second difference image RC2 is elastically deformed such that the nipple region N2 matches the nipple region N1. As described above, by elastically deforming the first difference image RC1 or the second difference image RC2 such that the X coordinates and the Y coordinates of the nipple region N1 and the nipple region N2 match each other, the determination accuracy of the BPE symmetry is further improved.

It should be noted that the resize processing unit 56 may detect the nipple region from the first low-energy image LE1 and the second low-energy image LE2 or the first high-energy image HE1 and the second high-energy image HE2, instead of the first difference image RC1 or the second difference image RC2.

### [Sixth Modification Example]

The sixth modification example is a modification example of the resize processing. In the fifth modification example, the size of the breast M shown in the first difference image RC1 or the second difference image RC2 is resized based on the coordinates of the nipple. In the present modification example, the size of the breast M is resized based on a length of a breast end shown in the first difference image RC1 or the second difference image RC2.

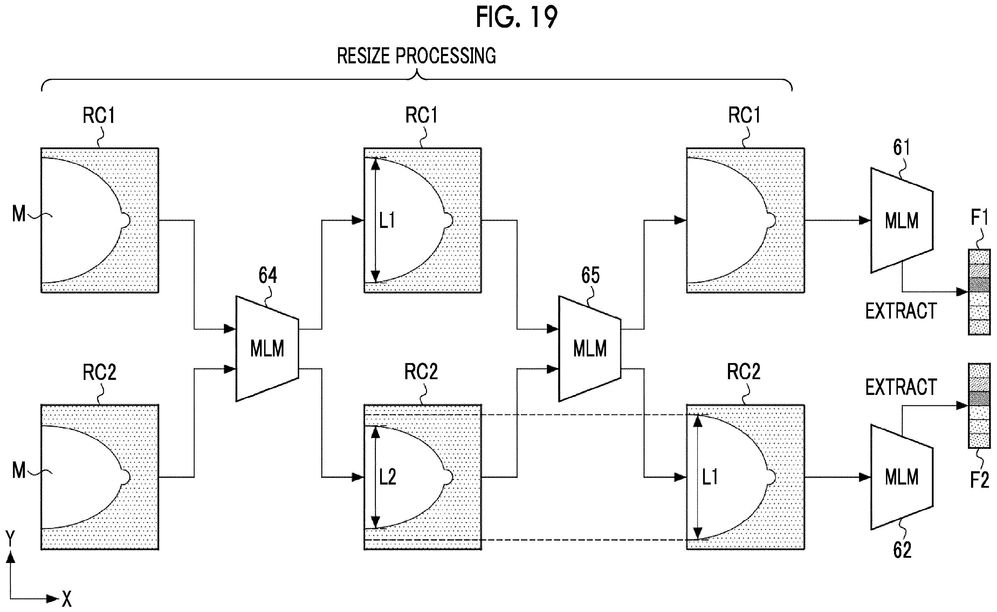

Fig. 19 conceptually shows the resize processing according to the sixth modification example. In the present modification example, the resize processing unit 56 detects the length of the breast end on the side opposite to the nipple, and resizes the size of the breast M shown in the first difference image RC1 or the second difference image RC2 based on the detected length of the breast end.

In the present modification example, the fourth MLM 64 detects a length L1 of the breast end of the breast M shown in the first difference image RC1 and a length L2 of the breast end of the breast M shown in the second difference image RC2. The fifth MLM 65 deforms the first difference image RC1 or the second difference image RC2 such that the length L1 of the breast end and the length L2 of the breast end match each other. In the example shown in Fig. 19, the fifth MLM 65 elastically deforms or resizes the second difference image RC2 such that the length L2 of the breast end matches the length L1 of the breast end, and outputs the second difference image RC2 after the deformation and the first difference image RC1.

According to the present modification example, as in the fourth modification example and the fifth modification example, the difference in the size of the breast M between the first difference image RC1 and the second difference image RC2 is reduced, so that the determination accuracy of the BPE symmetry is improved.

It should be noted that the resize processing unit 56 may detect the lengths L1 and L2 of the breast end from the first low-energy image LE1 and the second low-energy image LE2 or the first high-energy image HE1 and the second high-energy image HE2, instead of the first difference image RC1 or the second difference image RC2.

### [Seventh Modification Example]

The seventh modification example is different from the above-described embodiment in that the BPE determination processing is performed by using the first low-energy image LE1 and the second low-energy image LE2 that are acquired by the contrast-enhanced imaging processing, in addition to the first difference image RC1 and the second difference image RC2.

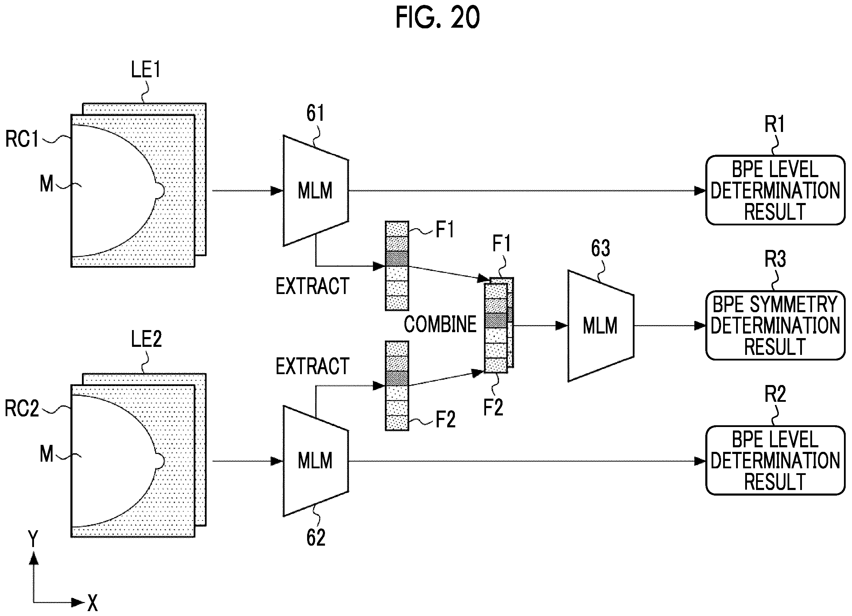

Fig. 20 schematically shows the BPE determination processing according to the seventh modification example. In the present modification example, the BPE determination processing unit 54 inputs the first difference image RC1 and the first low-energy image LE1 to the first MLM 61, and inputs the second difference image RC2 and the second low-energy image LE2 to the second MLM 62. For example, the BPE determination processing unit 54 combines the first difference image RC1 and the first low-energy image LE1 in the channel direction to input the combined image to the first MLM 61, and combines the second difference image RC2 and the second low-energy image LE2 in the channel direction to input the combined image to the second MLM 62.

In the present modification example, the first MLM 61 outputs the determination result R1 of the BPE level by performing the extraction of the feature value and the class classification based on the input first difference image RC1 and the first low-energy image LE1. The second MLM 62 performs the extraction of the feature value and the class classification based on the input second difference image RC2 and the second low-energy image LE2 to output the determination result R2 of the BPE level.

The mammary gland structure is clearly shown in the first low-energy image LE1 and the second low-energy image LE2. Therefore, by performing the BPE determination processing by using the first low-energy image LE1 and the second low-energy image LE2 in addition to the first difference image RC1 and the second difference image RC2, the BPE level and the BPE symmetry can be determined with higher accuracy.

It should be noted that the BPE determination processing may be performed by using the first high-energy image HE1 and the second high-energy image HE2, in addition to the first difference image RC1 and the second difference image RC2. In addition, the BPE determination processing may be performed by using the first low-energy image LE1 and the first high-energy image HE1 and the second low-energy image LE2 and the second high-energy image HE2, in addition to the first difference image RC1 and the second difference image RC2.

### [Eighth Modification Example]

The eighth modification example is different from the above-described embodiment in that mammary gland information detection processing is performed before the BPE determination processing.

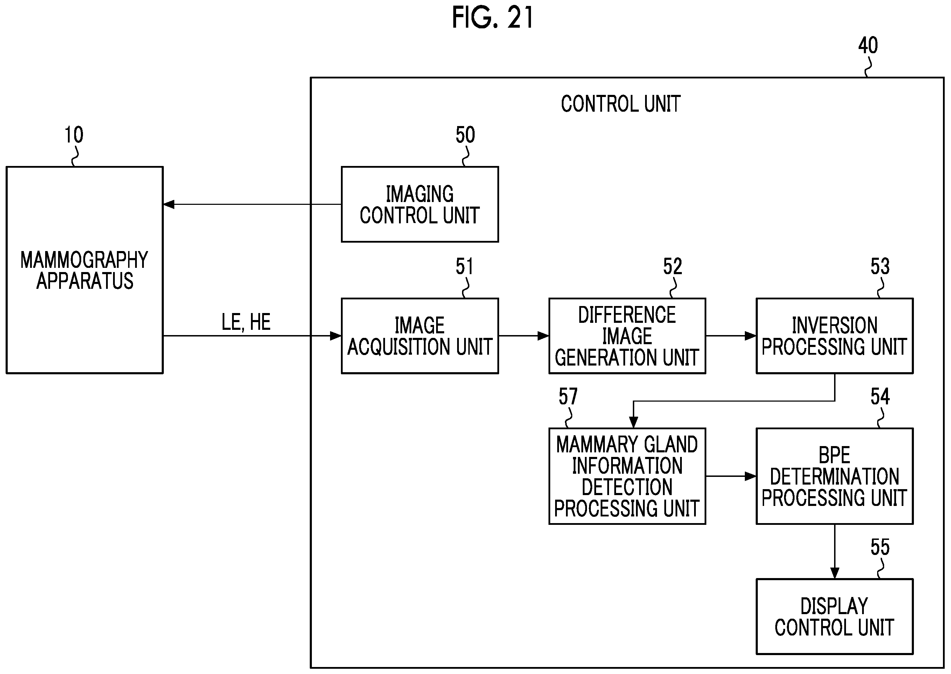

Fig. 21 shows functions implemented by the control unit 40 according to the eighth modification example. The control unit 40 according to the eighth modification example functions as a mammary gland information detection processing unit 57, in addition to the imaging control unit 50, the image acquisition unit 51, the difference image generation unit 52, the inversion processing unit 53, the BPE determination processing unit 54, and the display control unit 55. The control unit 40 performs the mammary gland information detection processing before performing the BPE determination processing and after performing the inversion processing.

The mammary gland information detection processing unit 57 performs the mammary gland information detection processing of acquiring first mammary gland information and second mammary gland information from the first low-energy image LE1 and the second low-energy image LE2, respectively. In the present modification example, the BPE determination processing unit 54 performs the BPE determination processing by using the first mammary gland information and the second mammary gland information in addition to the first difference image RC1 and the second difference image RC2.

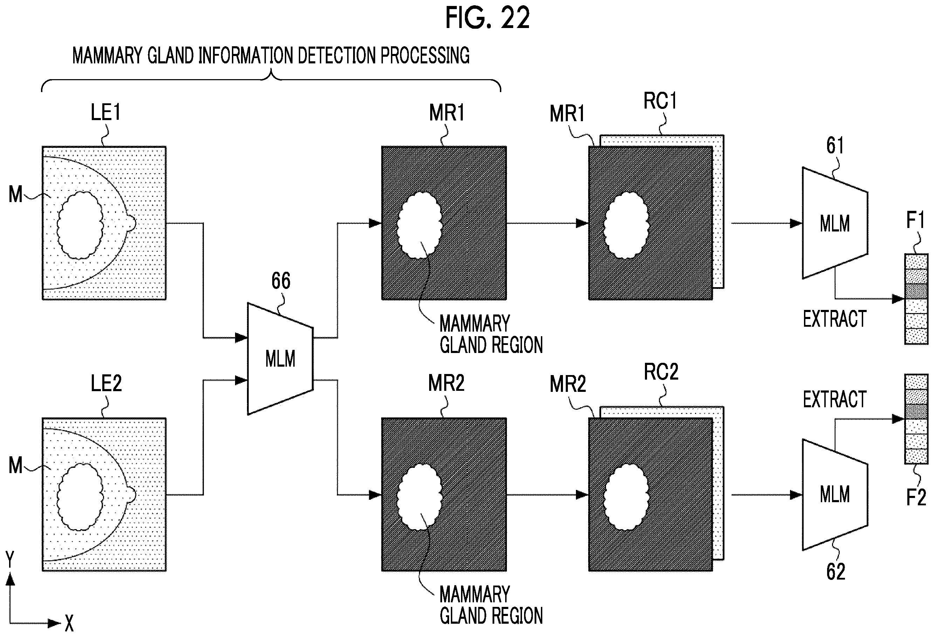

Fig. 22 conceptually shows the mammary gland information detection processing via the mammary gland information detection processing unit 57. The mammary gland information detection processing unit 57 detects a region including the mammary gland structure (hereinafter, referred to as a mammary gland region) from each of the first low-energy image LE1 and the second low-energy image LE2 by inputting the first low-energy image LE1 and the second low-energy image LE2 to a sixth MLM 66 that functions as a mammary gland region detection unit. The sixth MLM 66 is configured by, for example, a U-net, and specifies the mammary gland region via segmentation.

In the present modification example, the sixth MLM 66 outputs a first mammary gland region image MR1 representing the mammary gland region in the first low-energy image LE1, and a second mammary gland region image MR2 representing the mammary gland region in the second low-energy image LE2. The first mammary gland region image MR1 is an example of first mammary gland information. The second mammary gland region image MR2 is an example of second mammary gland information.

In the present modification example, the BPE determination processing unit 54 inputs the first difference image RC1 and the first mammary gland region image MR1 to the first MLM 61, and inputs the second difference image RC2 and the second mammary gland region image MR2 to the second MLM 62. For example, the BPE determination processing unit 54 combines the first difference image RC1 and the first mammary gland region image MR1 in the channel direction to input the combined image to the first MLM 61, and combines the second difference image RC2 and the second mammary gland region image MR2 in the channel direction to input the combined image to the second MLM 62.

In the present modification example, the first MLM 61 outputs the determination result R1 of the BPE level by performing the extraction of the feature value and the class classification based on the input first difference image RC1 and the first mammary gland region image MR1. The second MLM 62 performs the extraction of the feature value and the class classification based on the input second difference image RC2 and the second mammary gland region image MR2 to output the determination result R2 of the BPE level.

The mammary gland structure is clearly shown in the first low-energy image LE1 and the second low-energy image LE2, so that the mammary gland region can be accurately specified. Therefore, by performing the BPE determination processing by using the first mammary gland region image MR1 and the second mammary gland region image MR2 in addition to the first difference image RC1 and the second difference image RC2, the BPE level and the BPE symmetry can be determined with higher accuracy.

It should be noted that the mammary gland information detection processing unit 57 may detect the first mammary gland information and the second mammary gland information from the first high-energy image HE1 and the second high-energy image HE2 instead of the first low-energy image LE1 and the second low-energy image LE2. Further, the mammary gland information detection processing unit 57 may detect the first mammary gland information and the second mammary gland information from the first high-energy image HE1 and the second high-energy image HE2 in addition to the first low-energy image LE1 and the second low-energy image LE2. That is, the BPE determination processing unit 54 may perform the BPE determination processing by using the first mammary gland information and the second mammary gland information detected from the first low-energy image LE1 and the second low-energy image LE2, and the first mammary gland information and the second mammary gland information detected from the first high-energy image HE1 and the second high-energy image HE2, in addition to the first difference image RC1 and the second difference image RC2.

### [Ninth Modification Example]

In the eighth modification example, the mammary gland region is detected as the mammary gland information, but in the ninth modification example, a mammary gland volume is detected for each pixel as the mammary gland information.

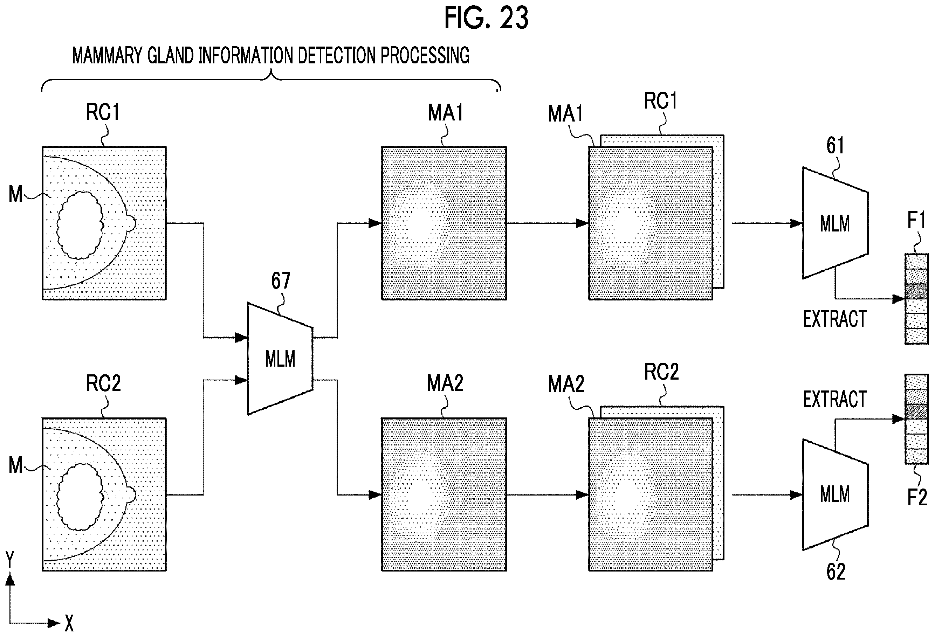

Fig. 23 conceptually shows the mammary gland information detection processing according to the ninth modification example. In the present modification example, the mammary gland information detection processing unit 57 detects the mammary gland volume for each pixel for each of the first low-energy image LE1 and the second low-energy image LE2 by inputting the first low-energy image LE1 and the second low-energy image LE2 to a seventh MLM 67 that functions as a mammary gland volume detection unit.

In the present modification example, the seventh MLM 67 outputs a first mammary gland volume image MA1 representing the mammary gland volume for each pixel of the first low-energy image LE1 and a second mammary gland volume image MA2 representing the mammary gland volume for each pixel of the second low-energy image LE2. The first mammary gland volume image MA1 is an example of first mammary gland information. The second mammary gland volume image MA2 is an example of second mammary gland information.

In the ninth modification example, processing other than the generation of the first mammary gland volume image MA1 instead of the first mammary gland region image MR1 and the generation of the second mammary gland volume image MA2 instead of the second mammary gland region image MR2 is the same as in the eighth modification example. In the present modification example, the BPE level and the BPE symmetry can also be determined with higher accuracy.

### [Other Modification Examples]

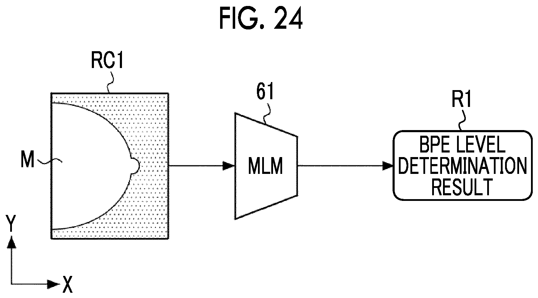

In the above-described embodiment and respective modification examples, the BPE determination processing unit 54 performs the determination of the BPE level and the BPE symmetry, but may perform only the determination of the BPE level. In addition, the BPE determination processing unit 54 may determine only the BPE level of one of the left breast M or the right breast M. For example, as shown in Fig. 24, the BPE determination processing unit 54 may determine the BPE level of one breast M by inputting the first difference image RC1 to the first MLM 61.

In addition, in the above-described embodiment and respective modification examples, the first MLM 61 and the second MLM 62 that function as the BPE level determination unit are each configured by the CNN, but are not limited to the CNN, and need only be configured by a neural network capable of extracting a feature value. That is, each of the first MLM 61 and the second MLM 62 need only be configured by machine learning-based neural networks, such as a CNN and a multilayer perceptron (MLP) neural network.

In addition, in the above-described embodiment and respective modification examples, the BPE determination processing unit 54 determines the BPE symmetry by using the third MLM 63 configured by the neural network, but the BPE symmetry may be determined by rule-based determination processing without using the neural network.

In the above-described embodiment and respective modification examples, the first MLM 61 and the second MLM 62 are the same model, but the first MLM 61 and the second MLM 62 may be different models. The different models refer to machine learned models in which training data used for machine learning is different, or machine learned models having different configurations such as the number of layers.

In addition, in the above-described embodiment and respective modification examples, the first MLM 61 and the second MLM 62 classify the BPE level into four classes and output the BPE level, but the number of classes to be classified is not limited to four and can be appropriately changed. Further, the first MLM 61 and the second MLM 62 may be configured to output a numerical value representing the BPE level.

The above-described embodiment and respective modification examples can be combined as appropriate as long as there is no contradiction. For example, the display control unit 55 may display the BPE region specified in the second modification example and the BPE asymmetric region specified in the fourth modification example in an overlaid manner on the first difference image RC1 and the second difference image RC2 or the first low-energy image LE1 and the second low-energy image LE2. The overlaid display includes display a plurality of regions in different colors. In addition, the display control unit 55 may selectively display the BPE region and the BPE asymmetric region in accordance with the input operation of the user with respect to the operation unit 44.

In addition, the control unit 40 may accumulate information related to the contrast-enhanced imaging, information on the person under an examination on which the contrast-enhanced imaging is performed, and the like in the storage unit 42 or the like as data. For example, the control unit 40 may accumulate information such as an injection start time point of the contrast agent into the breast M, an imaging time point of the contrast-enhanced imaging (or an elapsed time from the start of the injection of the contrast agent to the imaging time point), a thickness of the breast M, imaging conditions (a tube voltage and the like), and other patient information (age, menstrual cycle, presence or absence of menopause, and the like) in the storage unit 42 or the like.

In addition, in the above-described embodiment and respective modification examples, as a hardware structure of a processing unit that executes various kinds of processing, such as the imaging control unit 50, the image acquisition unit 51, the difference image generation unit 52, the inversion processing unit 53, the BPE determination processing unit 54, the display control unit 55, the resize processing unit 56, and the mammary gland information detection processing unit 57, various processors shown below can be used.

The various processors include a graphics processing unit (GPU) as well as a CPU. Further, the various processors include, in addition to a general-purpose processor which executes software (program) and functions as various processing units, such as a CPU, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application-specific integrated circuit (ASIC).

One processing unit may be configured by one of the various processors or may be configured by combining two or more processors of the same type or different types (for example, by combining a plurality of FPGAs or combining a CPU and an FPGA). Further, a plurality of the processing units may be configured by one processor.

A first example of the configuration in which the plurality of processing units are configured by one processor is a form in which one processor is configured by combining one or more CPUs and the software and this processor functions as the plurality of processing units, as represented by computers such as a client and a server. A second example is a form of using a processor that implements the function of the entire system including the plurality of processing units via one integrated circuit (IC) chip, as represented by a system on a chip (SoC) or the like. In this way, as the hardware structure, the various processing units are configured by using one or more of the various processors described above.

Further, the hardware structure of the various processors is, more specifically, an electric circuit (circuitry) in which circuit elements such as semiconductor elements are combined.

In addition, in the above-described embodiment and respective modification examples, the aspect has been described in which the program 42A is stored in the storage unit 42 in advance, but the present disclosure is not limited to this. The program 42A may be provided in a form of being recorded in a non-transitory recording medium such as a compact disc read only memory (CD-ROM), a digital versatile disc read only memory (DVD-ROM), or a universal serial bus (USB) memory. Further, the program 42A may be downloaded from an external apparatus via a network.

The above-described contents and the above-shown contents are detailed descriptions of portions related to the present disclosed technology and are merely examples of the present disclosed technology. For example, the description of the configuration, the function, the operation, and the effect are the description of examples of the configuration, the function, the operation, and the effect of the parts according to the present disclosed technology. Therefore, it goes without saying that unnecessary parts may be deleted, new elements may be added, or replacements may be made with respect to the above-described contents and the above-shown contents within a range that does not deviate from the gist of the present disclosed technology. Further, the description of, for example, common technical knowledge that does not need to be particularly described to enable the implementation of the present disclosed technology is omitted in the above-described contents and the above-shown contents in order to avoid confusion and to facilitate the understanding of the portions related to the present disclosed technology.

All of the documents, the patent applications, and the technical standards described in the present specification are incorporated into the present specification by reference to the same extent as in a case in which the individual documents, patent applications, and technical standards are specifically and individually stated to be described by reference.

The following technology can be understood from the above description.

### [Supplementary Note 1]

An information processing apparatus comprising: at least one processor, in which the processor is configured to: generate a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generate a second difference image representing a difference between a second low-energy image captured by irradiating the other of the left breast or the right breast with the radiation having the first energy and a second high-energy image captured by irradiating the other of the left breast or the right breast with the radiation having the second energy; determine an enhancement level of background mammary gland parenchyma of the one of the left breast or the right breast based on the first difference image; determine an enhancement level of background mammary gland parenchyma of the other of the left breast or the right breast based on the second difference image; and determine symmetry of enhancement regions of the background mammary gland parenchyma related to the left and right breasts based on a first feature value extracted from the first difference image and a second feature value extracted from the second difference image.

### [Supplementary Note 2]

The information processing apparatus according to supplementary note 1, in which the processor is configured to: determine the enhancement level by inputting the first difference image to a first machine learned model; determine the enhancement level by inputting the second difference image to a second machine learned model; extract a feature value generated by the first machine learned model as the first feature value; and extract a feature value generated by the second machine learned model as the second feature value.

### [Supplementary Note 3]

The information processing apparatus according to supplementary note 2, in which the first machine learned model and the second machine learned model are the same model.

### [Supplementary Note 4]

The information processing apparatus according to supplementary note 2 or 3, in which the processor is configured to: determine the symmetry by combining the first feature value and the second feature value and inputting the combined feature value to a third machine learned model.

### [Supplementary Note 5]

The information processing apparatus according to supplementary note 4, in which the processor is configured to: combine the first feature value and the second feature value in a channel direction and input the combined feature value to the third machine learned model.

### [Supplementary Note 6]

The information processing apparatus according to any one of supplementary notes 2 to 5, in which each of the first machine learned model and the second machine learned model classifies the enhancement level into a plurality of classes.

### [Supplementary Note 7]

The information processing apparatus according to supplementary note 4 or 5, in which the third machine learned model performs classification into two classes of symmetric and asymmetric.

### [Supplementary Note 8]

The information processing apparatus according to any one of supplementary notes 2 to 5, in which the first machine learned model specifies the enhancement region via segmentation from the first difference image and displays the specified enhancement region on the first difference image, and the second machine learned model specifies the enhancement region via segmentation from the second difference image and displays the specified enhancement region on the second difference image.

### [Supplementary Note 9]

The information processing apparatus according to any one of supplementary notes 2 to 5, in which the first machine learned model detects the enhancement region from the first difference image and displays a bounding box including the enhancement region on the first difference image, and the second machine learned model detects the enhancement region from the second difference image and displays a bounding box including the enhancement region on the second difference image.

### [Supplementary Note 10]

The information processing apparatus according to supplementary note 4 or 5, in which the third machine learned model specifies an asymmetric region in which the enhancement regions are not symmetric between the first difference image and the second difference image based on the first feature value and the second feature value and displays the specified asymmetric region.

### [Supplementary Note 11]

The information processing apparatus according to any one of supplementary notes 1 to 10, in which the processor is configured to: detect a nipple from the first difference image and the second difference image, the first low-energy image and the second low-energy image, or the first high-energy image and the second high-energy image, perform resizing of sizes of the left and right breasts shown in the first difference image and the second difference image based on coordinates of the detected nipple, and then determine the enhancement level and the enhancement region.

### [Supplementary Note 12]

The information processing apparatus according to any one of supplementary notes 1 to 10, in which the processor is configured to: detect a length of a breast end from the first difference image and the second difference image, the first low-energy image and the second low-energy image, or the first high-energy image and the second high-energy image, perform resizing of sizes of the left and right breasts shown in the first difference image and the second difference image based on the detected length of the breast end, and then determine the enhancement level and the enhancement region.

### [Supplementary Note 13]

The information processing apparatus according to supplementary note 1, in which the processor is configured to: determine the enhancement level by inputting the first difference image and the first low-energy image to a first machine learned model; determine the enhancement level by inputting the second difference image and the second low-energy image to a second machine learned model; extract a feature value generated by the first machine learned model as the first feature value; and extract a feature value generated by the second machine learned model as the second feature value.

### [Supplementary Note 14]

The information processing apparatus according to supplementary note 1, in which the processor is configured to: detect first mammary gland information from the first low-energy image and/or the first high-energy image; detect second mammary gland information from the second low-energy image and/or the second high-energy image; determine the enhancement level by inputting the first difference image and the first mammary gland information to a first machine learned model; determine the enhancement level by inputting the second difference image and the second mammary gland information to a second machine learned model; extract a feature value generated by the first machine learned model as the first feature value; and extract a feature value generated by the second machine learned model as the second feature value.

### [Supplementary Note 15]

The information processing apparatus according to supplementary note 14, in which each of the first mammary gland information and the second mammary gland information is a mammary gland region image representing a mammary gland region.

### [Supplementary Note 16]

The information processing apparatus according to supplementary note 14, in which each of the first mammary gland information and the second mammary gland information is a mammary gland volume image representing a mammary gland volume for each pixel.

### [Supplementary Note 17]

The information processing apparatus according to any one of supplementary notes 1 to 16, in which the processor is configured to: perform inversion processing of inverting one of the first difference image or the second difference image and then determine the enhancement level and the enhancement region.

## Claims

1. An information processing apparatus comprising:
at least one processor,
wherein the processor is configured to:
generate a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generate a second difference image representing a difference between a second low-energy image captured by irradiating the other of the left breast or the right breast with the radiation having the first energy and a second high-energy image captured by irradiating the other of the left breast or the right breast with the radiation having the second energy;
determine an enhancement level of background mammary gland parenchyma of the one of the left breast or the right breast based on the first difference image;
determine an enhancement level of background mammary gland parenchyma of the other of the left breast or the right breast based on the second difference image; and
determine symmetry of enhancement regions of the background mammary gland parenchyma related to the left and right breasts based on a first feature value extracted from the first difference image and a second feature value extracted from the second difference image.

2. The information processing apparatus according to claim 1,
wherein the processor is configured to:
determine the enhancement level by inputting the first difference image to a first machine learned model;
determine the enhancement level by inputting the second difference image to a second machine learned model;
extract a feature value generated by the first machine learned model as the first feature value; and
extract a feature value generated by the second machine learned model as the second feature value.

3. The information processing apparatus according to claim 2,
wherein the first machine learned model and the second machine learned model are the same model.

4. The information processing apparatus according to claim 2,
wherein the processor is configured to:
determine the symmetry by combining the first feature value and the second feature value and inputting the combined feature value to a third machine learned model.

5. The information processing apparatus according to claim 4,
wherein the processor is configured to:
combine the first feature value and the second feature value in a channel direction and input the combined feature value to the third machine learned model.

6. The information processing apparatus according to claim 2,
wherein each of the first machine learned model and the second machine learned model classifies the enhancement level into a plurality of classes.

7. The information processing apparatus according to claim 4,
wherein the third machine learned model performs classification into two classes of symmetric and asymmetric.

8. The information processing apparatus according to claim 2,
wherein the first machine learned model specifies the enhancement region via segmentation from the first difference image and displays the specified enhancement region on the first difference image, and
the second machine learned model specifies the enhancement region via segmentation from the second difference image and displays the specified enhancement region on the second difference image.

9. The information processing apparatus according to claim 2,
wherein the first machine learned model detects the enhancement region from the first difference image and displays a bounding box including the enhancement region on the first difference image, and
the second machine learned model detects the enhancement region from the second difference image and displays a bounding box including the enhancement region on the second difference image.

10. The information processing apparatus according to claim 4,
wherein the third machine learned model specifies an asymmetric region in which the enhancement regions are not symmetric between the first difference image and the second difference image based on the first feature value and the second feature value and displays the specified asymmetric region.

11. The information processing apparatus according to claim 1,
wherein the processor is configured to:
detect a nipple from the first difference image and the second difference image, the first low-energy image and the second low-energy image, or the first high-energy image and the second high-energy image, perform resizing of sizes of the left and right breasts shown in the first difference image and the second difference image based on coordinates of the detected nipple, and then determine the enhancement level and the enhancement region.

12. The information processing apparatus according to claim 1,
wherein the processor is configured to:
detect a length of a breast end from the first difference image and the second difference image, the first low-energy image and the second low-energy image, or the first high-energy image and the second high-energy image, perform resizing of sizes of the left and right breasts shown in the first difference image and the second difference image based on the detected length of the breast end, and then determine the enhancement level and the enhancement region.

13. The information processing apparatus according to claim 1,
wherein the processor is configured to:
determine the enhancement level by inputting the first difference image and the first low-energy image to a first machine learned model;
determine the enhancement level by inputting the second difference image and the second low-energy image to a second machine learned model;
extract a feature value generated by the first machine learned model as the first feature value; and
extract a feature value generated by the second machine learned model as the second feature value.

14. The information processing apparatus according to claim 1,
wherein the processor is configured to:
detect first mammary gland information from the first low-energy image and/or the first high-energy image;
detect second mammary gland information from the second low-energy image and/or the second high-energy image;
determine the enhancement level by inputting the first difference image and the first mammary gland information to a first machine learned model;
determine the enhancement level by inputting the second difference image and the second mammary gland information to a second machine learned model;
extract a feature value generated by the first machine learned model as the first feature value; and
extract a feature value generated by the second machine learned model as the second feature value.

15. The information processing apparatus according to claim 14,
wherein each of the first mammary gland information and the second mammary gland information is a mammary gland region image representing a mammary gland region.

16. The information processing apparatus according to claim 14,
wherein each of the first mammary gland information and the second mammary gland information is a mammary gland volume image representing a mammary gland volume for each pixel.

17. The information processing apparatus according to claim 1,
wherein the processor is configured to:
perform inversion processing of inverting one of the first difference image or the second difference image and then determine the enhancement level and the enhancement region.

18. An information processing method comprising:
generating a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generating a second difference image representing a difference between a second low-energy image captured by irradiating the other of the left breast or the right breast with the radiation having the first energy and a second high-energy image captured by irradiating the other of the left breast or the right breast with the radiation having the second energy;
determining an enhancement level of background mammary gland parenchyma of the one of the left breast or the right breast based on the first difference image;
determining an enhancement level of background mammary gland parenchyma of the other of the left breast or the right breast based on the second difference image; and
determining symmetry of enhancement regions of the background mammary gland parenchyma related to the left and right breasts based on a first feature value extracted from the first difference image and a second feature value extracted from the second difference image.

19. A program causing a computer to execute a process comprising:
generating a first difference image representing a difference between a first low-energy image captured by irradiating one of a left breast or a right breast, in which a contrast agent is injected, with radiation having first energy and a first high-energy image captured by irradiating the one of the left breast or the right breast with radiation having second energy higher than the first energy and generating a second difference image representing a difference between a second low-energy image captured by irradiating the other of the left breast or the right breast with the radiation having the first energy and a second high-energy image captured by irradiating the other of the left breast or the right breast with the radiation having the second energy;
determining an enhancement level of background mammary gland parenchyma of the one of the left breast or the right breast based on the first difference image;
determining an enhancement level of background mammary gland parenchyma of the other of the left breast or the right breast based on the second difference image; and
determining symmetry of enhancement regions of the background mammary gland parenchyma related to the left and right breasts based on a first feature value extracted from the first difference image and a second feature value extracted from the second difference image.
